(19) Europäisches Patentamt
European Patent Office
Office européen des brevets

(11) Veröffentlichungsnummer: **0 372 383**
**A1**

# (12) EUROPÄISCHE PATENTANMELDUNG

(21) Anmeldenummer: **89122065.9**

(22) Anmeldetag: **29.11.89**

(51) Int. Cl.5: **C12N 1/20, C12P 15/00, C07C 49/753, A61K 31/12**

Der Anmelder hat eine Erklärung nach Regel 28 (4) EPÜ (Herausgabe einer Probe nur an einen Sachverständigen) eingereicht. Eingangsnummer(n) der Hinterlegung(en): DSM 4769

(30) Priorität: **01.12.88 DE 3840519**

(43) Veröffentlichungstag der Anmeldung: **13.06.90 Patentblatt 90/24**

(84) Benannte Vertragsstaaten: **BE CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Wink, Joachim, Dr.**
**Bieberer Strasse 133**
**D-6050 Offenbach(DE)**
Erfinder: **Hammann, Peter, Dr.**
**Mörikestrasse 6**
**D-6233 Kelkheim(Taunus)(DE)**
Erfinder: **Kluge, Heinz, Dr.**
**Am Alten Birnbaum 10a**
**D-6238 Hofheim am Taunus(DE)**
Erfinder: **Roth, Alfred**

**verstorben(DE)**
Erfinder: **Winkler, Irvin, Dr.**
**In den Eichen 40**
**D-6237 Liederbach(DE)**

## (54) Neue Angucyclinone aus Streptomyceten, Verfahren zu ihrer Herstellung und ihre Verwendung.

(57) Angucyclinone aus Streptomyceten der Formel I

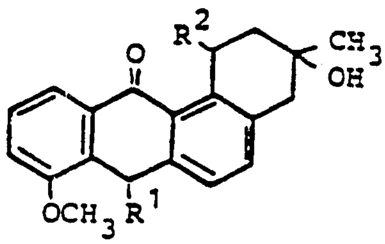

in der
a) $R^1$ und $R^2$ Hydroxyl,
b) $R^1$ eine Oxogruppe und $R^2$ Hydroxyl oder
c) $R^1$ und $R^2$ eine Oxogruppe
sind.
Verfahren zu ihrer Herstellung und ihre Verwendung.

EP 0 372 383 A1

## Neue Angucyclinone aus Streptomyceten, Verfahren zu ihrer Herstellung und ihre Verwendung

Es ist bekannt, daß Streptomyces spec. unter herkömmlichen Kulturbedingungen ein Angucyclinon mit Namen Ochromycinon synthetisiert [Bowie J.H., Johnson A.W. Tetrahedron Letters 16, 1449 (1967)]

Es wurde nun überraschend gefunden, daß Streptomyces spec. DSM 4769 neue Angucyclinone bildet.

Die Erfindung betrifft somit:

1. Eine Verbindung der allgemeinen Formel I,

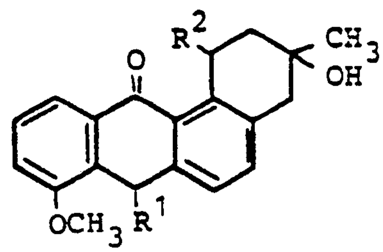

in der
a) $R^1$ und $R^2$ Hydroxyl,
b) $R^1$ eine Oxogruppe und $R^2$ Hydroxyl oder
c) $R^1$ und $R^2$ eine Oxogruppe
sind.

2. Ein Verfahren zur Herstellung der Verbindung der allgemeinen Formel I, das dadurch gekennzeichnet ist, daß Streptomyces spec. DSM 4769 kultiviert wird, bis sich die Verbindung der allgemeinen Formel I im Kulturmedium anhäuft und diese gegebenenfalls isoliert wird.

3. Die Verwendung der Verbindung der allgemeinen Formel I als therapeutisch wirksamer Stoff.

Die Erfindung wird im folgenden detailliert beschrieben, insbesondere in ihren bevorzugten Ausführungsformen. Ferner wird die Erfindung in den Patentansprüchen definiert.

Die Verbindung der allgemeinen Formel I kann mit Hilfe von Streptomyces spec. DSM 4769 hergestellt werden. Der Stamm wurde am 26.08.1988 bei der Deutschen Sammlung von Mikroorganismen und Zellkulturen unter der angegebenen Nummer nach den Bedingungen des Budapester Vertrages hinterlegt.

Streptomyces spec. DSM 4769 hat folgende charakteristische Merkmale:
Sporenfarbe: rot
Sporenkette: enge Spiralen
Sporenoberfläche: glatt
Melaninbildung: positiv

Anstelle von Streptomyces spec. DSM 4769 können auch dessen Mutanten nund Varianten verwendet werden, soweit sie eben die Verbindung der allgemeinen Formel I herstellen können. Solche Mutanten können in an sich bekannter Weise durch physikalische Mittel, beispielsweise Bestrahlung, wie mit Ultraviolett- oder Röntgenstrahlen oder chemische Mutagene, wie beispielsweise Ethylmethansulfonat (EMS), N-Methyl-N′-nitro-N-nitroso-guanidin (MNNG) oder 2-Hydroxy-4-methoxy-benzophenon (MOB) erzeugt werden.

Als bevorzugte Kohlenstoffquellen für die aerobe Fermentation eignen sich assimilierbare Kohlenhydrate und Zuckeralkohole, wie Glucose, Lactose oder D-Mannit, sowie kohlenhydrathaltige Naturprodukte, wie Malzextrakt. Als bevorzugte stickstoffhaltige Nährstoffe kommen in Betracht: Aminosäuren, Peptide und Proteine sowie deren Abbauprodukte, wie Peptone oder Tryptone, ferner Fleischextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate. Außerdem kann die Nährlösung beispielsweise Chloride, Carbonate, Sulfate oder Phosphate der Alkali-oder Erdalkalimetalle, Eisen, Zink und Mangan als zusätzliche anorganische Salze enthalten.

Die Bildung der Verbindung der allgemeinen Formel I verläuft besonders gut in einer Nährlösung, die Glycerin in Konzentrationen von 0,5 bis 6 %, bevorzugt 2 bis 4 %, sowie Sojamehl in Konzentrationen von 0,1 bis 4 %, bevorzugt 0,5 bis 2 % enthält, jeweils bezogen auf das Gewicht der gesamten Nährlösung.

Die Fermentation erfolgt aerob, also beispielsweise submers unter Schütteln oder Rühren in Schüttelkolben oder Fermentern, gegebenenfalls unter Einführen von Luft oder Sauerstoff. Die Fermentation kann in einem Temperaturbereich von etwa 18 bis 40° C, vorzugsweise bei etwa 25 bis 30° C, insbesondere bei 28

bis 30° C erfolgen. Man kultiviert den Mikroorganismus unter den genannten Bedingungen bis die stationäre Phase erreicht ist, etwa 60 bis 120 Stunden, bevorzugt 70 bis 75 Stunden.

Vorteilhaft kultiviert man in mehreren Stufen, d.h. man stellt zunächst eine oder mehrere Vorkulturen in einem flüssigen Nährmedium her, die dann in das eigentliche Produktionsmedium, die Hauptkultur, beispielsweise im Volumenverhältnis 1:10, überimpft werden. Die Vorkultur erhält man beispielsweise, indem man ein versportes Mycel in eine Nährlösung überimpft und etwa 48 bis 72 Stunden wachsen läßt. Das versporte Mycel kann erhalten werden, indem man den Stamm etwa 7 Tage auf einem festen oder flüssigen Nährboden, beispielsweise Hefe-Malz-Agar, wachsen läßt.

Der Fermentationsverlauf kann anhand des pH-Wertes der Kultur oder des Mycelvolumens, durch Dünnschichtchromatographie oder Ausprüfen der biologischen Aktivität überwacht werden.

Die Angucyclinone der allgemeinen Formel I liegen in der Kulturbrühe vor. Daher wird zweckmäßig für die Isolierung der Substanz das Mycel von der Kulturbrühe getrennt, beispielsweise durch Filtrieren oder Zentrifugieren. Die Verbindung der allgemeinen Formel I kann dann aus dem Überstand isoliert werden, zweckmäßig im pH-Bereich von 2 bis 8, vorzugsweise bei pH-Werten von 5 bis 7. Der Stoff kann mit herkömmlichen Mitteln, beispielsweise polaren Lösungsmitteln extrahiert werden, z.B. niederen Alkanolen. Es ist jedoch vorteilhaft, die Flüssigkeit über ein Adsorberharz, wie beispielsweise solche auf Polystyrolbasis, zu geben. Die Elution kann dann mit einem polaren Lösungsmittel erfolgen, bevorzugt niedrige Alkanole, wie z.B. Methanol, die möglicherweise noch mit Wasser vermischt werden. Aus dem Eluat kann das Lösungsmittel durch Destillation entfernt und der wäßrige, die Angucyclinone enthaltende Rückstand, getrocknet werden.

Die Verbindungen b und c der Formel I können nicht nur mikrobiologisch, sondern auch chemisch aus der Verbindung Ia gewonnen werden. Dazu löst man die Verbindung Ia in Lösungsmitteln, wie Chloroform, Methylenchlorid, Tetrahydrofuran, Essigester oder ($C_1$ bis $C_4$)-Alkoholen und rührt an der Luft mit oder ohne Luftzugabe unter Lichtausschluß 1 bis 20 Tage. Die Reaktionsdauer ist dabei abhängig von der Luftmenge im Reaktionsansatz. Als Endprodukt erhält man die Verbindung Ib. In einer Folgereaktion kann die Verbindung der Formel Ic durch Bestrahlung der erhaltenen Reaktionslösung in einem Quarzkolben mit UV-Licht (366 nm) über einen Zeitraum von 12 bis 48 Stunden hergestellt werden.

Die Angucyclinone der allgemeinen Formel I sind farblose amorphe Feststoffe, die gut in Methanol, Aceton, DMSO, Dioxan und Chloroform löslich sind, jedoch nicht in Wasser und Alkanen. Die Verbindungen der allgemeinen Formel I können entsprechend ihrer Stabilität in galenische Zubereitungen eingearbeitet werden. Die antibakterielle und antivirale Wirkung sowie die Wirkung gegen Protozoen kann im Agar-Diffusionstest bzw. durch Zellkulturtests in vitro gezeigt werden. Die Verbindungen zeigen insbesondere gegen Staphylococcus aureus und Staphylococcus pyogenes sowie gegen Adenoviren, HSVI- und HSVII-Viren und auch Trichomonas vaginalis (Protozoen) eine besonders gute Wirkung.

In den folgenden Beispielen wird die Erfindung weitergehend erläutert. Prozentangaben beziehen sich, wie schon in der vorangegangenen Beschreibung, auf das Gewicht.

**Beispiele:**

1. a) Herstellung einer Sporensuspension des Produzentenstammes:

100 ml Nährlösung (4 g Hefeextrakt, 10 g Malzextrakt, 4 g Glukose, 1 l Leitungswasser, pH-Wert vor dem Sterilisieren 7,3) in einem 500 ml Erlenmeyerkolben werden mit dem Stamm DSM 4769 beimpft und 72 Stunden bei 27° C und 120 UpM auf der rotierenden Schüttelmaschine inkubiert. Anschließend werden 20 ml Kulturflüssigkeit in einem 500 ml Erlenmeyerkolben mit dem Nährboden der obengenannten Zusammensetzung, dem 20 g Agar/l zur Verfestigung zugegeben wurde, gleichmäßig verteilt und dekantiert. Die Kulturen werden 10 bis 14 Tage bei 27° C inkubiert. Die nach dieser Zeit entstandenen Sporen eines Kolbens werden mit 500 ml entionisiertem Wasser, das einen Tropfen eines handelsüblichen nichtionischen Tensids (Triton X100, Fa. Serva) enthält, abgeschwemmt, sofort weiterverwendet oder bei -22° C aufbewahrt.

b) Herstellung einer Kultur bzw. Vorkultur des Produzentenstammes im Erlenmeyerkolben

Ein 500 ml Erlenmeyerkolben mit 100 ml einer Nährlösung der Zusammensetzung 2 % Fleischmehl, 10 % Malzextrakt, 1 % Calciumcarbonat und Wasser ad 100 % (pH 7,2 vor dem Autoklavieren) wird mit einer

auf einem Schrägröhrchen gezogenen Kultur oder mit 0,2 ml Sporensuspension von DSM 4769 angeimpft und auf einer Schüttelmaschine bei 120 UpM und 27 °C inkubiert. Die maximale Antibiotikumsproduktion ist nach 72 Stunden erreicht. Zum Animpfen von 10 und 100 l Fermentern genügt eine 48 Stunden alte Submerskultur (5 %) aus der gleichen Nährlösung.

2. Herstellung der Angucyclinone

Ein 10 l Fermenter angeimpft mit DSM 4769 wird unter folgenden Bedingungen betrieben:
Nährmedium:
30 g/l Glycerin
2 g/l Caseinpepton
1 g/l $K_2HPO_4$
1 g/l NaCl
0,5 g/l $MgSO_4 \cdot 7H_2O$
5 ml/l Spurenelementlösung
Spurenelemente:
3 g/l $CaCl_2 \cdot 2H_2O$
1 g/l $FeC_6O_7H_5$
0,2 g/l $MnSO_4$
0,1 g/l $ZnCl_2$
0,025 g/l $CuSO_4 \cdot 5H_2O$
0,02 g/l $Na_2B_4O_7 \cdot 10H_2O$
0,004 g/l $CoCl_2$
0,01 g/l $Na_2MoO_4 \cdot 2H_2O$
Inkubationszeit: 72 Stunden
Inkubationstemperatur: 30 °C
Rührergeschwindigkeit: 250 UpM
Belüftung: 4 l Luft/Min.

Durch wiederholte Zugabe weniger Tropfen ethanolischer Polyollösung kann die Schaumentwicklung unterdrückt werden. Das Produktionsmaximum wird nach ca. 70 Stunden (pH = 5,3) erreicht.

<u>3.a Isolierung der Angucyclinone</u>

8 l Fermenterinhalt werden filtriert und das Filtrat anschließend mit 3 x 6 l Methylenchlorid extrahiert. Nach dem Einrotieren im Vakuum wird der verbleibende Syrup (4g) an Kieselgel chromatographiert (Elutionsmittel $CHCl_3$:MeOH 30:1). Man erhält die Verbindung der Formel I

| | | |
|---|---|---|
| a) mit | $R^1$ -OH $R^2$ -OH | 330 mg |
| b) mit | $R^1$ =O $R^2$ -OH | 220 mg |
| c) mit | $R^1$ =O $R^2$ =O | 220 mg |

<u>b) Darstellung der Verbindung der Formel I mit $R^1$ =O und $R^2$ -OH</u>

50 mg der Verbindung der Formel I mit $R^1$ -OH und $R^2$ -OH werden in 10 ml Methylenchlorid unter Lichtausschluß an der Luft für 24 Tage gerührt. Das Lösungsmittel wird im Vakuum eingedampft und der Rückstand an Kieselgel chromatographiert (Elutionsmittel $CHCl_3$/MeOH:30/1). Man erhält 42 mg der gewünschten Verbindung.

c) Darstellung der Verbindungen b und c der Formel I:

100 mg der Verbindung der Formel Ia mit $R^1$ OH und $R^{2\ OH}$ werden in $CHCl_3$ (technisch) in einem Quarzkolben mit UV-Licht (366 nm) 24 Stunden bestrahlt. Nach dem Einrotieren wird der Rückstand an Kieselgel chromatographiert (Elutionsmittel $CHCl_3$/MeOH:30/1). Man erhält 7,3 mg der Verbindung Ib mit $R^1$ =O und $R^2$ -OH und 53 mg der Verbindung Ic mit $R^1$ =O und $R^2$ =O.

| Verbindung der Formel I | | | |
|---|---|---|---|
| $^{13}C$-NMR in $CDCl_3$ / $^1H$-NMR in $CDCl_3$ | | | |
| | a | b | c |
| | $R^1$ -OH | $R^1$ =O | $R^1$ =O |
| C | $R^2$ -OH | $R^2$ -OH | $R^2$ =O |
| 1 | 65,7 | 196,7 | 5,4 |
| 2 | 44,1 | 53,8 | 2,2 |
| 3 | 69,0 | 77,15 | |
| 4 | 45,2 | 44,1 | 2,9 |
| 4A | 136,4 | 146,4 | |
| 5 | 135,7 | 133,6 | 7,5 |
| 6 | 128,2 | 135,2 | 8,2 |
| 6A | 137,2 | 135,0 | |
| 7 | 63,3 | 181,1 | |
| 7A | 129,4 | 120,3 | |
| 8 | 156,8 | 159,9 | |
| 9 | 114,8 | 117,3 | 7,3 |
| 10 | 129,5 | 129,8 | 7,7 |
| 11 | 120,2 | 119,5 | 7,9 |
| 11A | 143,4 | 137,7 | |
| 12 | 187,5 | 184,3 | |
| 12A | 128,2 | 135,1 | |
| 12B | 140,0 | 134,3 | |
| 13 | 29,1 | 29,7 | 1,5 |
| 14 | 56,0 | 56,4 | 4,05 |

## Ansprüche

1. Die Verbindung der allgemeinen Formel I

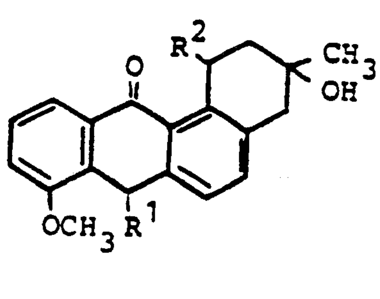

in der
a) $R^1$ und $R^2$ Hydroxyl,
b) $R^1$ eine Oxogruppe und $R^2$ Hydroxyl oder
c) $R^1$ und $R^2$ eine Oxogruppe

sind.

2. Verfahren zur Herstellung der Verbindung der allgemeinen Formel I nach Anspruch 1, dadurch gekennzeichnet, daß Streptomyces spec. DSM 4769 oder dessen Mutanten und Varianten kultiviert werden, bis sich die genannte Verbindung in der Kultur anhäufen und diese gegebenenfalls isoliert werden.

3. Verfahren nach Anspruch 2, dadurch gekennzeichnet, daß der Mikroorganismus in einer Nährlösung kultiviert wird, die 0,5 bis 6 % Glycerin sowie 0,1 bis 4 % Sojamehl enthält, jeweils bezogen auf das Gewicht der gesamten Nährlösung.

4. Verfahren nach Anspruch 3, dadurch gekennzeichnet, daß die Nährlösung 2 bis 4 % Glycerin sowie 0,5 bis 2 % Sojamehl enthält.

5. Verfahren nach einem oder mehreren der Ansprüche 2 bis 4, dadurch gekennzeichnet, daß die Kultivierung in einem Temperaturbereich von etwa 18 bis 40 °C durchgeführt wird.

6. Verwendung der Verbindung der allgemeinen Formel I nach Anspruch 1 als therapeutisch wirksamer Stoff.

7. Verwendung nach Anspruch 6, dadurch gekennzeichnet, daß die Verbindung der allgemeinen Formel I als Antibiotikum eingesetzt wird.

8. Verwendung nach Anspruch 7, dadurch gekennzeichnet, daß das Antibiotikum gegen Bakterien, Viren und Protozoen eingesetzt wird.

9. Streptomyces spec. DSM 4769, dessen Varianten und Mutanten, sofern sie die Verbindung der allgemeinen Formel I nach Anspruch 1 synthetisieren.

10. Verfahren zur Herstellung der Verbindungen Ib und c nach Anspruch 1, dadurch gekennzeichnet, daß man

a) die Verbindung Ia nach Anspruch 1 in einem Lösungsmittel löst, unter Lichtausschluß inkubiert und die Verbindung Ib erhält und

b) den unter a) erhaltenen Reaktionsansatz mit UV-Licht bestrahlt und die Verbindung Ic erhält.

Europäisches Patentamt

# EUROPÄISCHER RECHERCHENBERICHT

Nummer der Anmeldung

EP 89 12 2065

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | THE JOURNAL OF ANTIBIOTICS, Band 41, Nr. 9, September 1988, Seiten 1260-1264, Tokyo, JP; Y. SHIGIHARA et al.: "6-Deoxy-8-O-Methylrabelomycin and 8-O-Methylrabelomycin from a Streptomyces species" * Insgesamt * --- | 1-8,10 | C 12 N 1/20<br>C 12 P 15/00<br>C 07 C 49/753<br>A 61 K 31/12 |
| A | THE JOURNAL OF ANTIBIOTICS, Band 35, Nr. 12, Dezember 1982, Seiten 1627-1631, Tokyo, JP; H. MAEHR et al.: "Microbial products. VI Five novel metabolites related to Benz[a]anthracene from an unidentified actinomycete designated X-14881" * Insgesamt * --- | 1,6-8 | |
| A | FR-A-2 100 783 (SQUIBB) * Patentansprüche * ----- | 1,6-8 | |
| | | | **RECHERCHIERTE SACHGEBIETE (Int. Cl.5)** |
| | | | C 07 C 49/00<br>C 12 P 15/00<br>C 07 C 49/00 |

Der vorliegende Recherchenbericht wurde für alle Patentansprüche erstellt

| Recherchenort | Abschlußdatum der Recherche | Prüfer |
|---|---|---|
| DEN HAAG | 12-02-1990 | HENRY J.C. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)